# EUROPEAN PATENT APPLICATION

(11) **EP 4 074 840 A1**
(43) Date of publication of application: **19.10.2022**
(21) Application number: 20897780.1
(22) Date of filing: 11.12.2020
(51) Int. Cl.: C12Q 1/686

(54) **PCR METHOD AND PCR KIT FOR INCREASING ALLELIC DISCRIMINATION**

(30) Priority: 11.12.2019 KR 20190164776
(71) Applicant: Genotech Corp., Daejeon 34113 (KR)
(72) Inventor: KIM, Jae Jong, Daejeon 34049 (KR); LIM, Si-Kyu, Daejeon 34083 (KR); PARK, In Kyung, Daejeon 34061 (KR); KYUNG, AYoung, Daejeon 34946 (KR); LEE, Bo Mi, Nonsan-si, Chungcheongnam-do 32999 (KR); RYU, Jeounghyun, Cheongju-si, Chungcheongbuk-do 28337 (KR); CHA, Sun Ho, Sejong 30150 (KR); BAEK, SeungWoo, Daejeon 34049 (KR)
(74) Representative: Rimini, Rebecca
(86) International application number: PCT/KR2020/018147
(87) International publication number: WO 2021/118288

(57) **Abstract**

The present invention relates to a method and a kit for detecting alleles of which the specificity and sensitivity in a DNA polymerase chain reaction (PCR), which is widely used for the detection of minor alleles such as single nucleotide polymorphisms or somatic mutations, are increased. More specifically, the present invention relates to a PCR-based method and kit for single nucleotide polymorphism (SNP) genotyping and somatic mutation detection, the method and kit adding a partially or fully double-stranded oligonucleotide for increasing discrimination to a PCR solution for selective amplification of alleles, so that PCR amplification is not affected when a primer 3' terminal base is complementary (3'-matched) to a template but PCR amplification is strongly inhibited when a 3' terminal base is not complementary (3'-mismatched).

## Description

### Technical Field

The present disclosure relates to a method for detection of minor alleles, such as single nucleotide polymorphism (SNP) or somatic mutation wherein the specificity and sensitivity of DNA polymerase chain reaction (PCR) is increased for the alleles. More specifically, the present disclosure relates to a PCR-based SNP genotyping/somatic mutation detection technique wherein a partial or entire duplex-forming discrimination-boosting oligonucleotide (used interchangeably with "dbOligo" in the description and drawings) is added to a PCR solution for selectively amplifying an allele and acts to strongly inhibit PCR amplification when the 3' terminal base of a primer is not complementary to a template (3'-mismatched) but has no influence on PCR amplification when the 3' terminal base of a primer is complementary to a template (3'-matched).

### Background Art

The investigation of genetic variations involved in diseases such as genetic disorders and cancers is essential for predicting cures, establishing treatment methods, and observing prognosis and relapse. In addition, the identification or discrimination of such variations is very useful for breeding and selecting species and identifying origins and cultivars in the agricultural fields.

Genetic variation can be caused by endogenous factors of living organisms or by environmental factors to which living organisms are exposed throughout their lifespan. The most common among genetic variations in the human bodies is single nucleotide polymorphism (SNP). SNPs will be elucidated as a representative of various genetic variations in the detailed description, below.

The most common, economically favorable, and convenient among the techniques used for discriminating SNPs that living organisms including humans retain is a gene amplification technology using a polymerase, that is, polymerase chain reaction (PCR). Particularly, quantitative real-time PCR (qPCR or real-time PCR) can estimate variation amounts in real time during PCR.

Real-time PCR, which is a qualitative and quantitative strategic detection technique for nucleic acids, finds applications in a variety of fields including health, agriculture, food, environment, etc. Since its development in early 1990, real-time PCR has been continuously studied to overcome technical limitations, developing into a more accurate and precise technique.

Particularly, when used to develop a gene discrimination kit, real-time PCR suffers from the representative technical limitations of generation of non-specific signals and low discrimination between mutants and wild-type gene sequences.

The control of non-specific signals is essential for the development of real-time PCR as a diagnostic technique particularly to detect cancers, pathogens, etc. False positives which are apt to appear due to non-specific signals degrade the reliability of the test. Diagnostic technologies that have to detect a trace amount of targets, such as non-invasive liquid biopsy, require enhanced PCR efficiency and specificity so as to accurately diagnose target variations present in trace amounts. As a rule, strategies for increasing the efficiency and specificity of PCR include creation of mixtures used in PCR, design of specific probes or primers, and enzyme modification.

Efficiency and specificity of PCR have been improved as follows.

### A. PCR mixture

Studies on PCR mixtures have been directed toward improving PCR reactions, diminishing unnecessary primer dimers, reducing generation of non-specific PCR products due to mismatches of primers with targets, and preventing PCR efficiency drops caused by high GC rates and the formation of specific higher structure. In this regard, additives for PCR mixtures have been suggested, as exemplified by dimethylsulfoxide (DMSO), betaine, etc. So-called "HotStart PCR" is implemented to block unwanted nonspecific amplifications caused by primers binding on unwanted target DNA at low temperatures.

Examples of "HotStart PCR" include the addition of a DNA polymerase (DNAP)-specific monoclonal antibody {Biotechniques (1994) 16(6):1134-1137}, the addition of an aptamer, which is a single-stranded oligonucleotides inhibitory of the activity of DNA polymerase {US/005693502A (1997); J. Mol. Biol. 271:100-111 (1997); Nucleic Acids Research Supplement No.3: 309-310 (2003)}, and the inhibition of non-specific DNA synthesis by using a double-stranded nucleotide that can bind to DNA polymerase at low temperatures to inhibit the activity of the DNA polymerase, but does not form a duplex at a specific temperature or higher in a PCR condition and thus has no influences on the activity of the DNA polymerase {J. Mol Biol. 264(2):268-278 (1996); US 8,043,816 B2 (2011)}.

### B. Enzyme engineering

Many DNA polymerases have been developed to block non-specific signals or enhance allele discrimination in PCR. On the whole, thermostable DNA polymerase is used in PCR. DNA polymerases can be divided into seven different families. Usually selected for PCR are thermostable DNA polymerases in family A, such as Taq DNA polymerase and in family B, such as Pfu DNA polymerase and a group of enzymes from archaea.

The family to which Taq DNA polymerase belong is characterized by 5'→3' nuclease (both exonuclease and endonuclease activity, also referred to as flap endonuclease or FEN1). Advantage is taken of this activity to release specific signals through the degradation of hydrolysis probes (TaqMan probes). Lacking 3'→5' exonuclease activity, Taq DNA polymerase is very suitable for the PCR that employs a primer having a 3'-mismatch with the template DNA. For an allele-specific (AS) primer with a 3'-mismatch or an amplification refractory mutation system (ARMS) primer, the amplification efficiency of a mutant gene, a wild-type gene, or the two alleles are determined according to the match or mismatch of the 3' base, with relatively favorable clinical outcomes resulting therefrom. For the most cases, however, amplification occurs in part even with 3' mismatched primers, occasionally causing discrimination errors in highly sensitive diagnosis for detecting mutations incorporated into the wild-type sequence.

Therefore, the discrimination of the enzyme between a match and a mismatch in the 3' terminus is very important for increasing specificity for SNP or mutation discrimination. In this regard, polymerases with improved ability to discriminate between matches and mismatches in 3' terminal sequences of allele-specific primers are reported {PLos One. 9(5): e96640 (2014); KR 10-2017-0088373 (2017); US 0034879A1 (2013); WO 082449A2 (2015); US 9267120B2 (2016)}.

### C. Employment of specific probe and primer

Specific probes and primers are used to specifically detect target amplicons. Probes with high specificity were developed to increase PCR efficiency and specificity. When applied to real-time PCR, DNA-binding fluorophores, such as SYBR green I and SYTO9, allows for detecting the entire amplicons, but cannot filter off non-specific signals. To overcome this problem, target sequence-specific probes have been developed. Examples of these probes include TaqMan probe (dual labelled signaling hydrolysis probe) {P Natl Acad Sci USA 88, 7276-280 (1991)}, molecular beacons {Methods. 25, 463-71 (2001)}, scorpion probes {Nat Biotechnol. 17, 804-07 (1999)}, LUX (light upon extension) primers {Nucleic acids research. 30, e137 (2002)}, and Amplifluor primers {BioTechniques. 26, 552-58 (1999)}.

In addition, development was made of primers or oligonucleotide blockers with high specificity for selectively amplifying target mutant genes.

Methods employing primers with high specificity may be exemplified by AS-PCR (allele specific PCR) that makes discrimination even one base difference in the 3' terminus and ARMS-PCR (amplification refractory mutation system PCR) that is designed to add an additional mutant sequence plus one mutant base to the 3' terminus {Mol Cell Probes. 18. 349-352 (2004); Nucleic Acids Res 17. 2503-2516 (1989); Nat Biotechnol. 17. 804-807 (1999); Cytokine 71, 278-282 (2015)}. In recent years, primers composed of two separate parts, such as the DPO (dual-priming oligonucleotide) of Seegene Inc. {J. Am. Chem. Soc. 126, 4550-4556 (2004); Biomol. Detect. Quantif. 1 3-7 (2014); J. Clin. Microbiol. 49. 3154-3162 (2011)} and myT primer of Swift Biosciences (http://www.swiftbiosci.com/technology/myt-primers), have been developed to discriminate mismatches while increasing annealing stability.

### <Related Art Document>

### <Patent literature>

Japanese Patent Number 2011-41572 A "Composition and method for enhanced sensitivity and selectivity of nucleic acid synthesis"
U. S. Patent Number 8,043,816 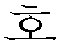 "Compositions and methods for temperature-dependent nucleic acid synthesis"
Korean Patent Number 10-2013-0138700 A "Compositions and methods for temperature-dependent nucleic acid synthesis"

### <Non-patent literature>

Kazunori Ikebukuro et al., Nucleic Acid Research Supplement No.3 309-310 "Screening of DNA Aptamers inhibiting Taq DNA Polymerase using algorithm mimicking evolution"
Yun Lin et al., J. Mol. Biol. (1997) 271, 100-111 "Inhibition of Multiple Thermostable DNA Polymerases by a Heterodimeric Aptamer"
Nick A. Rejali et al., Clinical Chemistry 64:5 000-000 (2018) "The Effect of Single Mismatches on Primer Extension"
Hao-Ching Wang et al., Biochemistry 2014, 53, 2865-2874 "DNA Mimic Proteins: Functions, Structures, and Bioinformatic Analysis"
BioTechniques 28:278-282 (February 2000) "Specificity-Enhanced Hot-Start PCR: Addition of Double-Stranded DNA Fragments Adapted to the Annealing Temperature"

### Detailed Description of the Invention

### Technical Problem

For cancer diagnosis, a mutated DNA accounting for cancer is very difficult to specifically detect from clinical samples because specimens such as biological tissues, blood, feces, saliva, and so on have a trace amount of the mutated gene among a vast amount of wild-type DNA. There is a need for a diagnostic method that guarantees the specificity allowing for detecting a mutation incorporated in a trace amount of 1 % or less into a vast amount of wild-type genes, alongside high robustness for clinical applications. Above all, low false-positive for wild-type sequences and high specificity for mutated sequences should be required. For these reasons, improved ingredients of PCR mixture, specific probes and/or primers, or enzyme modification has been developed to increase PCR efficiency and specificity for use in diagnosis necessary for high sensitivity, such as in cancer diagnosis.

When used, reagents for increasing amplification efficiency, such as DMSO, betaine, etc., monoclonal antibodies for inhibiting DNA polymerase, and oligonucleotides, so-called aptamers, inhibitory of DNA polymerase activity often increase DNA amplification efficiency and repress the amplification of non-specific PCR products or the formation of primer dimers, but have difficulty in increasing discrimination of alleles.

AS primers or ARMS primers, which are designed to discriminate alleles through the presence or absence of 3' mismatches according to mutated sequences of alleles, are generally employed to improve discrimination of alleles. When AS primers, designed to make a mismatch on only one base, are used, high PCR efficiency is obtained, but discrimination of alleles is poor. For ARMS primers that are designed to make mismatches on two or more bases, discrimination of alleles is higher compared to AS primers, but the PCR efficiency is poor, with the resultant reduction of a limit of detection (LOD) .

A mutant DNA polymerase with improved ability to discriminate 3' mismatches and matches is used to increases detection specificity for mutated sequences. However, such mutant DNA polymerases often decrease in enzymatic activity and thus become poor in detection sensitivity.

In order to effectively detect and diagnose alleles or mutated sequences, therefore, there is a need for a novel method that solves the problems with the related art and can highly discriminate matches and mismatches between a primer's 3' terminus and a template DNA.

### Technical Solution

The present disclose aims to provide a PCR buffer composition and a PCR method using same, which allow for discrimination of alleles according to a primer's 3' mismatch or match, thus exhibiting improved discrimination between and/specificity for alleles or mutated sequences.

To accomplish the aim, an aspect of the present disclosure provides a PCR buffer comprising a double helix forming oligonucleotide (discrimination-boosting oligonucleotide, "dbOligo"), and a PCR method using same.

In spite of the presence of a mismatch at the 3' terminus of a primer, AS-PCR or ARMS-PCR can be usually conducted. During a PCR reaction, the DNA polymerase may perform dNTP addition in a subsequent stage although there is a 3' mismatch. Once the polymerase breaks through the 3' mismatch, PCR can be smoothly carried out from the next round cycle of PCR because of no mismatches of template.

In order to maximize discrimination between two genes, therefore, PCR should be restrained in the initial step when the 3' terminal base of the primer makes a mismatch with the template. The frequent progression of PCR under 3' mismatch results from the frequent erroneous reaction attributed to the initial addition of an excess of DNA polymerase (abbreviated to "DNAP") for efficient PCR. In the presence of an excess of DNAP added initially, a primer (abbreviated to "P") is hybridized with a template (abbreviated to "T₂") to form a DNA (that is, P/T₂) with a 3' mismatch, and as a result, the concentration of a substrate and enzyme complex, that is, [P/T₂·DNAP] increases, provoking frequent 3' mismatched PCR reaction errors. The formation of [DNAP·P/T₂] complex can be reduced by decreasing initial enzyme input. At a low level of DNA polymerase, however, the exponential amplification of an amplicon cannot be expected. In light of the PCR feature that a closed system must be maintained for automatic processes and contamination prevention, it is very difficult to continually added a DNA polymerase during progression of the reaction.

In the present disclosure, a composition is designed to allow a DNA polymerase to be properly used in a PCR reaction.

In this regard, a composition particularly containing a nucleic acid, a protein, and an organic compound, which can bind reversibly to a DNA polymerase, is added to a PCR solution, a PCR kit, or a PCR master mix. More particularly, a substance that does not inhibit the activity of DNA polymerase during PCR may be contained. Proteins that can combine with DNA polymerase may be proteins structurally similar to DNA (DNA mimic proteins) (Biochemistry 53, 2865-2874 (2014)), and nucleic acids that combine with DNA polymerases may be duplex-forming oligonucleotides and more particularly, double-stranded DNA to which a DNA polymerase readily bind.

Leading to the present disclosure, intensive and thorough research conducted by the present inventors resulted in the finding that when set forth to have has a melting temperature (Tm) higher than the lowest temperature (annealing temperature) set in PCR steps, a double-stranded oligonucleotide or a fragment thereof, or a duplex-forming oligonucleotide in a PCR solution is less apt to participate in 3' mismatched PCR amplification and thus can increase discrimination between alleles or mutated genes and wild-type genes.

The present disclosure pertains to a PCR kit for detection of a mutant, the kit comprising:
(a) a forward primer and a reverse primer for at least one template including a target DNA sequence having a potential mutation locus;
(b) a DNA polymerase for DNA polymerization from the forward primer and the reverse primer which combine with the template; and
(c) one or more discrimination-boosting oligonucleotides which are each complementary to none of the template, the forward primer, and the reverse primer, can each reversibly combine with the DNA polymerase, and each form, in part or in entirety, a duplex.

In an embodiment of the present disclosure, the PCR kit for detection of a mutant may further comprise (d) at least one template including a target DNA sequence having a potential mutation locus.

In the PCR kit for detection of a mutant according to another embodiment of the present disclosure, the first base at the 3' terminus of the forward primer corresponds to the potential mutation locus of the target DNA sequence.

In the PCR kit for detection of a mutant according to another embodiment of the present disclosure, the forward primer is an allele-specific (AS) primer or an amplification refractory mutation system (ARMS) primer.

In the PCR kit for detection of a mutant according to another embodiment of the present disclosure, the discrimination-boosting oligonucleotide (dbOligo) may be at least one selected from among a DNA duplex, an RNA/DNA hybrid duplex, a double-stranded oligonucleotide, a partially or entirely complementary oligonucleotide single strand(s) capable of forming a partial or entire DNA duplex, a partially or entirely complementary oligonucleotide single strand(s) capable of forming a partial or entire DNA/RNA hybrid duplex, a partially or entirely complementary oligonucleotide single strand(s) capable of forming a partial or entire double-stranded oligonucleotide, and an oligonucleotide capable of forming a partial or perfect hairpin duplex. The discrimination-boosting oligonucleotide almost little interferes with PCR reactions and enhances discrimination of mutations such as SNP or somatic mutations. As for the discrimination-boosting oligonucleotide, the partially or entirely complementary oligonucleotide single strand(s) capable of forming a partial or entire duplex may be a self-complementary single strand(s) or two more oligonucleotide single strands including two or more partially or entirely complementary sequences.

In the PCR kit for detection of a mutant according to another embodiment of the present disclosure, the discrimination-boosting oligonucleotide has any sequence.

In the PCR kit for detection of a mutant according to another embodiment of the present disclosure, the mutation in the target DNA sequence is a single nucleotide polymorphism.

In the PCR kit for detection of a mutant according to another embodiment of the present disclosure, the DNA polymerase is a thermostable DNA polymerase.

In the PCR kit for detection of a mutant according to another embodiment of the present disclosure, the DNA polymerase is a wild-type or a mutant DNA polymerase.

In the PCR kit for detection of a mutant according to another embodiment of the present disclosure, the discrimination-boosting oligonucleotide has a length of 10 to 100 bases, 10 to 90 bases, 10 to 80 bases, 10 to 70 bases, or 10 to 60 bases, particularly 15 to 50 bases, 15 to 40 bases, or 15 to 30 bases (both inclusive). When the oligonucleotide is less than 10 bases or exceeds 100 bases in length, only an insignificant effect of enhancing discrimination of alleles is obtained.

In the PCR kit for detection of a mutant according to another embodiment of the present disclosure, the discrimination-boosting oligonucleotide has a Tm identical or greater than an annealing temperature set forth for PCR amplification. When the Tm of the discrimination-boosting oligonucleotide is lower than the annealing temperature, the discrimination of alleles is little enhanced.

In the PCR kit for detection of a mutant according to another embodiment of the present disclosure, the discrimination-boosting oligonucleotide has a Tm of 50-85°C. Given a range of 50-85°C, the Tm of the oligonucleotide can bring about a great improvement in discriminating alleles.

According to an embodiment of the present disclosure, the PCR kit or method employs a probe, capable of fluorescence resonance energy transfer, for the template or an amplicon-binding substance, such as SYBR Green I or is adapted to identifying an amplicon by general electrophoresis.

In another embodiment of the present disclosure, the kit for detection of a mutant further comprises a probe, capable of fluorescence resonance energy transfer, for the template, for example, a fluorescence resonance energy transfer probe modified with a reporter and a quencher.

In another embodiment of the present disclosure, the kit for detection of a mutant further comprises an amplicon-binding substance such as SYBR Green I.

Also, the present disclosure pertains to a method for detection of a genetic mutation, the method comprising the steps of:
(a) providing a template including a target DNA sequence having a potential mutation locus, a forward primer and a reverse primer both combining with the template, a DNA polymerase for polymerizing DNA from the forward and reverse primers, a discrimination-boosting oligonucleotides that is complementary to none of the template, the forward primer, and the reverse primer and can reversibly combine with the DNA polymerase, with no or little interference with activity of the DNA polymerase during a polymerase chain reaction;
(b) performing a polymerase chain reaction using the DNA polymerase in the presence of the template, the forward primer, the reverse primer, and the discrimination-boosting oligonucleotide; and
(c) acquiring an amplification curve from the reaction of step (b).

In an embodiment of the present disclosure, the method for detection of a genetic mutation further comprises a step of (d) determining from the amplification curve acquired in step (c) whether the target DNA sequence includes a mutation.

According to another embodiment of the present disclosure, the method further comprises providing a probe for the template in step (a) or (b).

According to another embodiment of the present disclosure, the method further comprises providing an amplicon-binding substance such as SYBR Green I in step (a) or (b).

In the method for detection of a genetic mutation according to another embodiment of the present disclosure, the first base at the 3' terminus of the forward primer corresponds to the potential mutation locus of the target DNA sequence.

In the method for detection of a genetic mutation according to another embodiment of the present disclosure, the forward primer is an allele-specific (AS) primer or an amplification refractory mutation system (ARMS) primer.

In the method for detection of a genetic mutation according to another embodiment of the present disclosure, the discrimination-boosting oligonucleotide (dbOligo) may be at least one selected from among a DNA duplex, an RNA/DNA hybrid duplex, a double-stranded oligonucleotide, a partially or entirely complementary oligonucleotide single strand(s) capable of forming a partial or entire DNA duplex, a partially or entirely complementary oligonucleotide single strand(s) capable of forming a partial or entire DNA/RNA hybrid duplex, a partially or entirely complementary oligonucleotide single strand(s) capable of forming a partial or entire double-stranded oligonucleotide, and an oligonucleotide capable of forming a partial or perfect hairpin duplex.

In the method for detection of a genetic mutation according to another embodiment of the present disclosure, the discrimination-boosting oligonucleotide includes arbitrary sequence.

In the method for detection of a genetic mutation according to another embodiment of the present disclosure, the mutation in the target DNA sequence is a single nucleotide polymorphism.

In the method for detection of a genetic mutation according to another embodiment of the present disclosure, the DNA polymerase is a thermostable DNA polymerase.

In the method for detection of a genetic mutation according to another embodiment of the present disclosure, the DNA polymerase is a wild-type or a mutant DNA polymerase. For use in PCR aiming to highly discriminate alleles, DNA polymerase has been modified through amino acid substitution, deletion and/or insertion. In the presence of such a mutant DNA polymerase, the discrimination of alleles can be further enhanced by employing the discrimination-boosting oligonucleotide of the present disclosure.

In the method for detection of a genetic mutation according to another embodiment of the present disclosure, the discrimination-boosting oligonucleotide has a length of 10 to 100 bases, 10 to 90 bases, 10 to 80 bases, 10 to 70 bases, or 10 to 60 bases, particularly 15 to 50 bases, 15 to 40 bases, or 15 to 30 bases.

In the method for detection of a genetic mutation according to another embodiment of the present disclosure, the discrimination-boosting oligonucleotide has a Tm identical or greater than an annealing temperature set forth for PCR amplification.

In the method for detection of a genetic mutation according to another embodiment of the present disclosure, the discrimination-boosting oligonucleotide has a Tm of 50-85°C.

According to an embodiment of the present disclosure, the PCR kit or the method employs a probe, capable of fluorescence resonance energy transfer, for the template or an amplicon-binding substance, such as SYBR Green I or is adapted to identifying an amplicon by general electrophoresis.

### Advantageous Effects

According to the kit or the method of the present disclosure, a remarkable improvement can be brought about in the specificity and sensitivity of PCR for detecting minor alleles such as single nucleotide polymorphisms or somatic mutations. In a PCR solution, the discrimination-boosting oligonucleotide acts to interfere with PCR amplification when the primer's 3' terminal base mismatches the template and to enhance PCR amplification when the primer's 3' terminal base matches the template, thereby remarkably enhancing the specificity and sensitivity, compared to the absence of the discrimination-boosting oligonucleotide.

The PCR kit or method employing a discrimination-boosting oligonucleotide according to the present disclosure remarkably enhances specificity and sensitivity in general PCR as well as real-time PCR.

The PCR kit or method employing a discrimination-boosting oligonucleotide according to the present disclosure can recruit a substance capable of detecting amplicons in addition to a hydrolysis probe generating a fluorescent signal, thus making it possible to easily identify the position and amplification of complementary or non-complementary mutation loci.

Therefore, the PCR kit or method of the present disclosure is advantageous for detecting alleles from a sample containing a mixture of trace amounts of various species.

Moreover, the PCR kit or method of the present disclosure can easily detect a mutant gene even if it is present at a trace amount in a sample.

### Brief Description of the Drawings

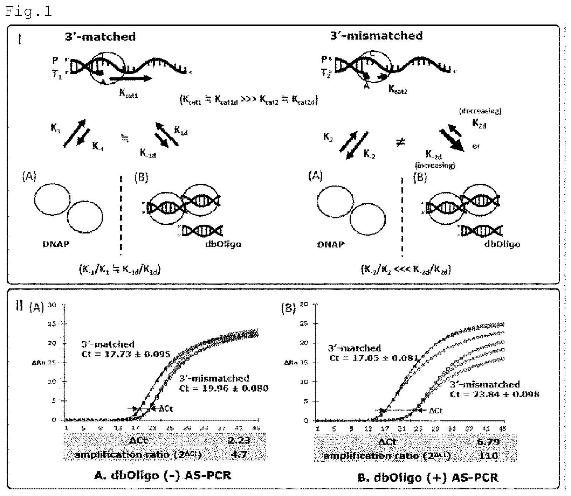

FIG. 1 shows a conceptual diagram elucidating the mechanism of PCR according to the presence or absence of a discrimination-boosting oligonucleotide ("dbOligo") (I) and amplification curves of AS-PCR obtained according to the presence or absence of dbOligo (II).
   (A) AS-PCR in the absence of dbOligo; (B) AS-PCR in the presence of dbOligo
      K_{cat1}, K₁, and K₋₁: reaction constants of enzyme in the corresponding steps for (A) in the absence of dbOligo when a 3'-matched primer is employed,
      K_{cat1d}, K_{1d}, and K_{-1d}: reaction constant of enzyme in the corresponding steps for (B) in the presence of dbOligo when a 3'-matched primer is employed,
      K_{cat2}, K₂, and K₋₂: reaction constants of enzyme in the corresponding steps for (A) in the absence of dbOligo when a 3'-mismatched primer is employed,
      K_{cat2d}, K_{2d}, and K_{-2d}: reaction constant of enzyme in the corresponding steps for (B) in the presence of dbOligo when a 3'-mismatched primer is employed.
      The dbOligo has the sequence of SEQ ID NO: 14 or 15 and is added in an amount of 20 pmol or not added (Test No. 1).
      Specific amplification ratio is calculated to be 2^{ΔCt},
      Specific amplification ratio = amplification of 3'matched DNA/amplification of 3'mismatched DNA.
FIG. 2 shows amplification curves elucidating discrimination ability of allele-specific PCR according to amounts of dbOligo.
   m, mutated template (matches the 3' terminus of the primer);
   w, wild-type template (mismatches the 3' terminus of the primer).
   The dbOligo used has the sequence of SEQ ID NO: 13 and is used in an amount of 0, 10, 20, 40, 60, or 80 pmol.
FIG. 3 shows amplification curves elucidating the effect of dbOligo on modified Taq DNA polymerase and wild-type Taq DNA polymerase. The dbOligo used has the sequence of SEQ ID NO: 13 and is added in an amount of 40 pmol.
   Wt-Taq, wild-type Taq DNA polymerase; Mut-Taq, modified (R536K) Taq DNA polymerase,
   m, mutated template (matches the 3' terminus of the primer);
   w, wild-type template (mismatches the 3' terminus of the primer).
FIG. 4 shows amplification curves and electrophoresis photographic images elucidating the effect of dbOligo in PCR without using a hydrolysis probe.

### Best Mode for Carrying Out the Invention

Unless otherwise defined, all technical and scientific terms used in this specification have the same meaning as those commonly understood by those skilled in the art. In general, the nomenclature used in this specification is well-known and commonly used in the art.

The present disclosure may be understood more readily by reference to the following detailed description taken in connection with the accompanying figures and examples, which form a part of this disclosure. It is to be understood that this disclosure is not limited to the specific devices, methods, applications, conditions or parameters described and/or shown herein, and that the terminology used herein is for the purpose of describing particular embodiments by way of example only and is not intended to be limiting of the claimed disclosure. As used in the specification including the appended claims, the singular forms "a," "an," and "the" include the plural, and reference to a particular numerical value includes at least that particular value, unless the context clearly dictates otherwise.

Unless otherwise stated, the embodiments of the present disclosure utilize molecular biological methods commonly known in the art to which the present disclosure belongs.

As used herein, the term "base" refers to a nucleobase and is intended to encompass canonical purine and pyrimidine bases including adenine, guanine, cytosine, uracil, and thymine, whether natural or synthetic, and their analogs and derivatives, but with no limitations thereto.

As used herein, the term "nucleotide" refers to a basic building block of nucleic acids, which is composed of a sugar, a base, and a phosphate group. The sugar, which is ribose or deoxyribose, has a base bonded to C-1' thereof and a phosphate group bonded to C-5' thereof. Here, the term "nucleotide" encompasses nucleotide analogs. The sugar may or may not be substituted by other structural analogs. Examples of nucleic acid analogs composed of such compounds include phosphorothioate DNA, PNA (peptide nucleic acid), phosphoramidate DNA, morpholino, and LNA (locked nucleic acid), but are not limited thereto.

As used herein, the terms "nucleic acid", "polynucleotide", "oligonucleotide", "oligomer" or other terms equivalent thereto are used to cover polymers of various monomers including polymers of monomers corresponding to nucleobase, i.e., polymers of monomers such as deoxyribonucleic acids, ribonucleic acids, phosphorothioate DNA, LNA (locked nucleic acid), PNA (peptide nucleic acid), etc., and polymers structurally similar thereto (e.g., morpholinos).

The term "oligonucleotide", as used herein, means a short polynucleotide. Usually, an oligonucleotide is about 250 nucleotides or less, about 200 nucleotides or less, or 100 nucleotides or less in length.

In the present disclosure, the "oligonucleotide" may include modified oligonucleotides. Here, the term "modification" indicates any modification on nucleobases (e.g., purine analogs, pyrimidine analogs, inverted base, methylated analogs, fluoro analogs, etc.), linking regions (linkers) of nucleosides (e.g., amino (NH₂) linker, carboxyl linker, thiol (SH) linker, etc.), the phosphate group, 5'-terminal, 3'-terminal, or internal bases of oligonucleotides, and combinations thereof.

As used herein, the term "template" means "template nucleic acid" which is used to set the genetic sequence of new strands during replication in PCR. All of DNA strands which are naturally present, naturally occur, and are artificially synthesized, fall within the scope of the template.

As used herein, the term "target" refers to a nucleic acid to be analyzed.

On the whole, a PCR buffer may contain non-essential components such as DMSO, betaine, an aptamer, or an antibody in addition to essential components such as Mg⁺⁺, and dNTP.

For PCR using a primer that allows for discrimination of two alleles according to the presence or absence of a 3' mismatch, a PCR buffer may contain a discrimination-boosting oligonucleotide selected from among a DNA duplex, an RNA/DNA hybrid duplex, a double-stranded oligonucleotide, a partially or entirely complementary oligonucleotide single strand(s) capable of forming a partial or entire DNA duplex, a partially or entirely complementary oligonucleotide single strand(s) capable of forming a partial or entire DNA/RNA hybrid duplex, a partially or entirely complementary oligonucleotide single strand(s) capable of forming a partial or entire double-stranded oligonucleotide, and an oligonucleotide capable of forming a partial or perfect hairpin duplex, so as to further increase discrimination of alleles (ΔCt or ΔCp, ΔCq).

In addition, the discrimination-boosting oligonucleotide used for PCR in the present disclosure may include oligonucleotides which are rendered to form a duplex by, for example, chemically coupling two or more single strands having partially or entirely complementary sequences through psoralen or a structurally similar compound thereto, or a chemical linker (e.g., disulfide linker, bismalemide linker, etc.), or by linking termini of the oligonucleotides or extending the oligonucleotide to form a complementary sequence, such as a hairpin loop structure, within single strands. Preferable among them is the formation of a complementary sequence within single strands of oligonucleotides whereby a duplex can be easily formed. This is because a duplex is more easily formed with a single strand, which is not dispersed in a buffer, has a partially or entirely self-complementary sequence, compared to two separate strands which have partially or entirely complementary sequences to each other. Therefore, it is advantageous for accomplishing the goal of the present disclosure to form a duplex by using a chemical method for preventing physical separation of two complementary sequences or a method for making a complementary sequence within a single strand.

The discrimination-boosting oligonucleotide of the present disclosure for a DNA polymerase may vary in binding affinity for DNA polymerase, depending on various factors including the base sequence thereof, kinds of specific nucleic acids, or oligonucleotide length. In the present disclosure, however, the binding affinity is not limited by specific base sequences, specific kinds of nucleic acids, or a specific range of oligonucleotide length.

When the complementary sequence accounting for the formation of a duplex is too long or when there are many kinds of complementary oligonucleotide pairs in a PCR solution, the formation of a duplex is reciprocally impeded or the formation of a non-specific duplex is caused during PCR. For instance, when a genomic DNA is denatured during PCR, the resulting single strands are too long to form an accurate duplex again, which makes it difficult to attain the goal of the present disclosure. Thus, the complementary sequence suitable for forming a duplex is preferably 10 bases in length and more preferably 15 to 50 bases in length, with no specific limitations imparted to the complementary sequence and kinds of nucleic acids.

In the present disclosure, a PCR solution may contain discrimination-boosting oligonucleotide in an amount of 0.01-1,000 pmol, 0.1-500 pmol, 0.1-400 pmol, 0.1-300 pmol, 0.1-200 pmol, 0.1-100 pmol, or 1-80 pmol per 20 µL of the PCR solution. However, the amount of the discrimination-boosting oligonucleotide may vary depending on the sequence of a target gene to be detected, a sample, and PCR conditions, and is not limited to specific concentration.

The present disclosure is applicable to PCR using a DNA polymerase, especially polymerases of family A (E. *coli* Pol I lineage). This polymerase may be selected from DNA polymerases derived from thermophilic bacteria, particularly thermophilic eubacteria, and more particularly *Thermus* spp., *Thermotoga* spp. *Thermococcus* spp., *Deinococcus* spp., and *Bacillus* spp.

In accordance with the present disclosure, a duplex of the discrimination-boosting oligonucleotide has a melting temperature (Tm) higher than an annealing temperature of general PCR. For instance, when a duplex of the discrimination-boosting oligonucleotide has a Tm lower than an annealing temperature in PCR, the discrimination-boosting oligonucleotide has difficulty in forming a duplex, leading to a decrease in binding affinity for DNA polymerase. Thus, the discrimination-boosting oligonucleotide used in the present disclosure is particularly configured to have a base sequence such that its duplex region has a Tm higher than an annealing temperature of general PCR.

The advantages of the present disclosure can be explained through the mechanism depicted in FIG. 1. This mechanism is provided to more accurately understand the present disclosure, but is not to be construed to perfectly describe the entirety of the present disclosure. However, even if the description of the mechanism of the present disclosure is not complete, the effect of the present disclosure should not be denied for this reason.

FIG. 1 elucidates the mechanism of PCR in terms of kinetic parameters when a primer's 3' terminus makes a match (left panel, "3'-matched") or a mismatch (right panel, "3'-mismatched") with a template. The 3'-matched and the 3'-mismatched are each subjected to two conditions: (A) PCR in the absence of a discrimination-boosting oligonucleotide (dbOligo); and (B) PCR in the presence of dbOligo. In the drawings, "DNAP" stands for DNA polymerase; "dbOligo" for an oligonucleotide that has any sequence and is added in a double-stranded form or forms a duplex in a reaction solution; "K₁" and "K₋₁" for kinetic parameters in a forward and a reverse direction, respectively, under the condition of making a match between a primer's 3' terminus and a template upon PCR in the absence of dbOligo; "K_{1d}" and "K_{-1d}" for kinetic parameters in a forward and a reverse diction, respectively, under the condition of making a match between a primer's 3' terminus and a template upon PCR in the presence of dbOligo; "K₂" and "K₋₂" for kinetic parameters in a forward and a reverse direction, respectively, under the condition of making a mismatch between a primer's 3' terminus and a template upon PCR in the absence of dbOligo; and "K_{2d}" and "K_{-2d}" for kinetic parameters in a forward and a reverse diction, respectively, under the condition of making a mismatch between a primer's 3' terminus and a template upon PCR in the presence of dbOligo.

According to the match or mismatch between a template and a primer's 3' terminus, the DNA synthesis process of a DNA polymerase (hereinafter, referred to as "DNAP") is very complicated. Various factors implicated in enzyme kinetics include a primer (hereinafter referred to as "P") serving as a substrate, a template DNA (hereinafter, 3'-matched template DNA is abbreviated to "T₁", 3'-mismatched template DNA is abbreviated to "T₂" for convenience), a hybrid thereof (P/T₁ or P/T₂), DNA polymerase (DNAP), dNTPs, Mg⁺⁺, and PPi.

Upon the formation of DNAP ·P/T complex, K_{cat} is greatly different according to the 3' match (DNAP ·P/T₁) or 3' mismatch (DNAP ·P/T₂). Reportedly, PCR very quickly starts when a primer's 3 terminus matches a template, compared to when a primer's 3' terminus mismatches a template {Clin Chem. 64(5) :801-809 (2018)}. According to the report, K_{cat}/Km is approximately 100 to 1000-fold higher for 3'-match than 3'-mismatch. This is because K_{cat} is substantially lowered (approximately 10- to 600-fold) and Km is slightly increased (up to 3-fold) for 3'-mismatch.

From the documents, it could be inferred that when a primer's 3' terminus matches a template, the DNAP ·P/T₁ complex, once formed, continually perform repeated cycles of polymerization with dNTPs without detachment of the enzyme on the basis of high K_{cat1} until completion of the polymerization whereas the DNAP ·P/T₂ complex formed for a mismatch between a primer's 3' terminus and a template frequently undergoes dissociation and association of the DNA polymerase due to low K_{cat2} (K_{cat1} >>> K_{cat2}).

In the presence of a double-stranded oligonucleotide according to the present disclosure, there is a significant difference in real-time PCR between 3' match and 3'-mismatch (amplification curves in II of FIG. 1).

For a 3' match, similar Ct values were obtained irrespective of the presence or absence of a dbOligo. In light of this fact, similar values are obtained in the reaction speed (K_{cat1} ≒ K_{cat1d}) and the dissociation and association ratio of DNA polymerase for template DNA (K-₁/K₁ ≒ K_{-1d}/K_{1d}) between the presence and the absence of dbOligo, leading to the inference that the dbOligo has almost no or little influences on the activity of the DNA polymerase.

The present inventors predicted that when a primer's 3' terminus mismatches a template, K_{cat2} and K_{cat2d}, although lower than K_{cat1} or K_{cat1d}, might be similar to each other irrespective of the presence or absence of a dbOligo (K_{cat2} ≒ K_{cat2d} <<< K_{cat1} ≒ K_{cat1d}). Contrary to the prediction, the presence of an dbOligo remarkably decreased the polymerization for 3' mismatch (3'-mismatched in FIG. 1(B)). The reason is that the DNA polymerase is inferred to decrease in association constant (K_{2d}) or increase in dissociation constant (K_{-2d}) for formation of DNAP ·P/T₂ in the presence of a dbOligo, compared to the absence of a dbOligo. That is to say, it is inferred that the addition of a dbOligo to a PCR solution might make a great change in the dissociation and association ratio of DNA polymerase for template DNA or product DNA (K₋₂/K₂ <<< K_{-2d}/K_{2d}). This difference of kinetic parameters upon the presence of a dbOligo increases discrimination of PCR between 3' match and 3' mismatch.

As a result, it is possible to explicitly discriminate real-time PCR amplification curves, depending on the 3' match or mismatch with alleles or mutant genes, so that the goal of the present disclosure can be attained.

In addition, when exponential amplification proceeds with the effective progression of real-time PCR on the 3'-matched template DNA, the synthesized DNA amplicon serves as a new template and as such, the amount of the enzyme substrate drastically increases. In an early stage of PCR, [P/T₁] ≒ [P/T₂] <<< [dbOligo]. During PCR amplification, [P/T₁] increases in an exponential manner, with the resultant disappearance of the predominance of [dbOligo] over [P/T₁] . Thus, PCR will be further smoothly conducted. In contrast, the predominance of [dbOligo] over [P/T₂] is kept going for 3' mismatch between a primer's 3 terminus and a DNA template, repressing PCR progression. Thus, discrimination on the amplification curves can be further increased.

### Mode for Carrying Out the Invention

A better understanding of the present disclosure may be obtained through the following examples that are set forth to illustrate, but are not to be construed to limit the present disclosure.

The PCR using a discrimination boosting oligonucleotide (dbOligo) to increase discrimination of alleles or mutated genes was designated *"STexS (SNP Typing with excellent specificity)"* PCR.

Below, a detailed description will be given of *"STexS"* PCR in conjunction with concrete examples.

### <PCR Condition>

Unless stated otherwise, PCR solutions and conditions used in Examples of the present disclosure are as follows.

Wild-type target template DNA and mutant target template DNA, forward primers, reverse primers, and hydrolysis probes for signal detection used in PCR are summarized in Table 1, below.

For use in detecting the variation of the target genes EGFR c.2369 C>T (p.T790M); EGFR c.2573 T>G (p.L858R) and BRAF c.1799 rc. A>T (p.V600E), the forward primers, called AS primers, are designed to match the mutated genes and mismatch 1 base pair of the wild-type genes at the 3' terminus of each of the primers.

Enzymes for each PCR were 2 units (0.05-0.08 µM) of Taq DNA polymerase (GenoTech). PCR buffer (10 mM Tris, pH 9.0, 1.5 mM MgCl₂, 60 mM KCl, 10 mM (NH₄)₂SO₄) amounted to a total of 20 µl. PCR was conducted using ABI 7500 Real-Time PCR System, starting at 95°C for 5 min followed by 45 cycles of 95°C for 30 seconds, 55°C for 40 seconds. Results are expressed as mean values of measurements for all trials that were tested in triplicate.

To confirm an improvement in discrimination upon PCR, various types of dbOligo including single-stranded DNA (SD), complementary double-stranded DNA (DD), and single-stranded DNA with an internal complementary sequence (hairpin DNA; HD) were added in an amount of 1 to 80 pmol according to test (Tables 2, 3, and 4).

Template DNAs were prepared by artificially synthesizing the sequences of SEQ ID NOS: 44, 45, 46, 47, 48, and 49, inserting the sequences into pTOP Blunt V2 (Enzynomics, Korea), transforming the plasmids into *E. coli,* and digesting the amplified plasmids with proper restriction enzymes. Before use, the purified plasmid DNAs were quantitated.

**TABLE 1**

| Target Sequence: EGFR c.2369 C>T (p.T790M) | | | |
|---|---|---|---|
| | Seq. ID | Amount | Note |
| Wild Template | 44 | 1 × 10⁷ copies | |
| Mutant Template | 45 | 1 × 10⁷ copies | |
| Forward Primer | 1 | 20 pmol | |
| Reverse Primer | 7 | 20 pmol | |
| Hydrolysate Probe | 10 | 10 pmol | dual labelled |

| Target Sequence: EGFR c.2573 T>G (p.L858R) | | | |
|---|---|---|---|
| | Seq. ID | Amount | Note |
| Wild Template | 46 | 1 × 10⁷ copies | |
| Mutant Template | 47 | 1 × 10⁷ copies | |
| Forward Primer | 5 | 20 pmol | |
| Reverse Primer | 8 | 20 pmol | |
| Hydrolysate Probe | 11 | 10 pmol | dual labelled |

| Target Sequence: BRAF c.1799 rc. A>T (p.V600E) | | | |
|---|---|---|---|
| | Seq. ID | Amount | Note |
| Wild Template | 48 | 1 × 10⁷ copies | |
| Mutant Template | 49 | 1 × 10⁷ copies | |
| Forward Primer | 6 | 20 pmol | |
| Reverse Primer | 9 | 20 pmol | |
| Hydrolysate Probe | 12 | 10 pmol | dual labelled |

### <EXAMPLE 1> Improvement of Discrimination Ability for 3'-Mismatch in Presence of dbOligo

After PCR using AS primers (forward primer: SEQ ID NO: 1, reverse primer: SEQ ID NO: 7) for targeting EGFR T790M, the discrimination ability ΔCt1 (ΔCt1 = Ct of mutated gene - Ct of wild-type gene) was given very low values 1.48 - 2.16 (Test Nos. 1-6 in Table 2).

When the complementary double-stranded DNA (DD) was added as an dbOligo in an amount of 20 pmol for PCR (Test No. 1 (SEQ ID NOS: 14/15) and Test No. 2 (SEQ ID NOS: 17/18, 19/20, 14/15, 21/22, 23/24, 25/26)), ΔCt2 (ΔCt2 = Ct of mutated gene in the presence of dbOligo - Ct of wild-type gene in the presence of dbOligo) was measured to 3.04 - 6.79, with ΔΔCt (ΔΔCt = ΔCt2-ΔCt1) amounting to 1.56 - 4.56, demonstrating a great improvement in discrimination ability (Test Nos. 1 and 2 in Table 2, panel II in Fig. 1 II; Test No. 1).

For test groups in which the single-stranded DNA (SD) having the same sequence as one strand of DD was added in an amount of 20 - 40 pmol (Test No. 4 (SEQ ID NO: 25 and SEQ ID NO: 26)), ΔCt2 was measured to be 2.28 - 2.82, with ΔΔCt amounting to 0.20 - 0.74, which was insignificant in discrimination ability, compared to the complementary double-stranded DNA (DD) groups [Test No. 4 (SEQ ID NOS: 25/26)], with ΔCt2 = 5.66 and ΔΔCt = 3.58 (Table 2). This result shows that the discrimination-boosting oligonucleotide (dbOligo) is very important in enhancing the discrimination ability.

In addition, all the tests with the hairpin DNA (HD), which forms a duplex due to its complementary base sequence within a single strand, were measured to have ΔCt2 of 2.46 - 5.99 and a ΔΔCt of 0.30 - 3.51, exhibiting a great improvement in discrimination ability (Test No. 1 (SEQ ID NO: 16) and Test No. 5 (SEQ ID NOs: 16, 27, 13, 28, 29, 30, 31)) (Table 2), like DD.

**TABLE 2**

| Test No. | dbOligo | | | ΔCt (Mutant Ct -Wild type Ct)*** | | ΔΔCt (ΔCt2 - ΔCt1) |
|---|---|---|---|---|---|---|
| | Seq. ID | Type, * Duplex No,** Tm*** | Amount (pmol) | ΔCt1^{#} | ΔCt2^{##} | |
| 1 | 14/15 | DD, 24, 68 | 20 | 2.23 | 6.79 | **4.56** |
| | 16 | HD, 24, 68 | | | 5.74 | **3.51** |
| 2 | 17/18 | DD, 20, 64 | 20 | 1.48 | 3.04 | **1.56** |
| | 19/20 | DD, 22, 66 | | | 5.31 | **3.83** |
| | 14/15 | DD, 24, 68 | | | 4.57 | **3.09** |
| | 21/22 | DD, 26, 71 | | | 4.02 | **2.54** |
| | 23/24 | DD, 28, 72 | | | 5.66 | **4.18** |
| | 25/26 | DD, 30, 74 | | | 5.70 | **4.22** |
| 3 | 25/26 | DD, 30, 74 | 10 | 1.57 | 4.58 | **3.01** |
| | | | 20 | | 5.67 | **4.10** |
| | | | 40 | | 7.26 | **5.69** |
| | | | 60 | | 8.26 | **6.69** |
| | | | 80 | | 15.07 | **13.5** |
| 4 | 25/26 | DD, 30, 74 | 20 | 2.08 | 5.66 | **3.58** |
| | 25 | SD, 30, - | | | 2.82 | **0.74** |
| | 26 | SD, 30, - | | | 2.46 | **0.38** |
| | 25 | SD, 30, - | 40 | | 2.28 | **0.20** |
| | 26 | SD, 30, - | | | 2.37 | **0.29** |
| 5 | 16 | HD, 24, 68 | 20 | 2.16 | 5.52 | **3.36** |
| | 27 | HD, 22, 66 | | | 5.52 | **3.36** |
| | 13 | HD, 20, 64 | | | 5.99 | **3.83** |
| | 28 | HD, 18, 60 | | | 5.68 | **3.52** |
| | 29 | HD, 16, 58 | | | 3.70 | **1.54** |
| | 30 | HD, 14, 54 | | | 3.15 | **0.99** |
| | 31 | HD, 12, 47 | | | 2.46 | **0.30** |
| 6 | 13 | HD, 20, 64 | 10 | 1.23 | 3.69 | **2.46** |
| | | | 20 | | 4.84 | **3.61** |
| | | | 40 | | 6.72 | **5.49** |
| | | | 60 | | 7.34 | **6.11** |
| | | | 80 | | 9.05 | **7.82** |

| | | | | | | |
|---|---|---|---|---|---|---|
| * dbOligo type: SD, single-stranded DNA; DD, double-stranded DNA; HD, hairpin structure DNA,** Duplex no: Number of bases in duplex, *** Tm: melting temperature of double-stranded oligonucleotide, # ΔCt1: ΔCt in the absence of dbOligo, ## ΔCt2: ΔCt in the presence of dbOligo. | | | | | | |

### <EXAMPLE 2> Discrimination Ability for 3'-Mismatch According to Concentration of dbOligo

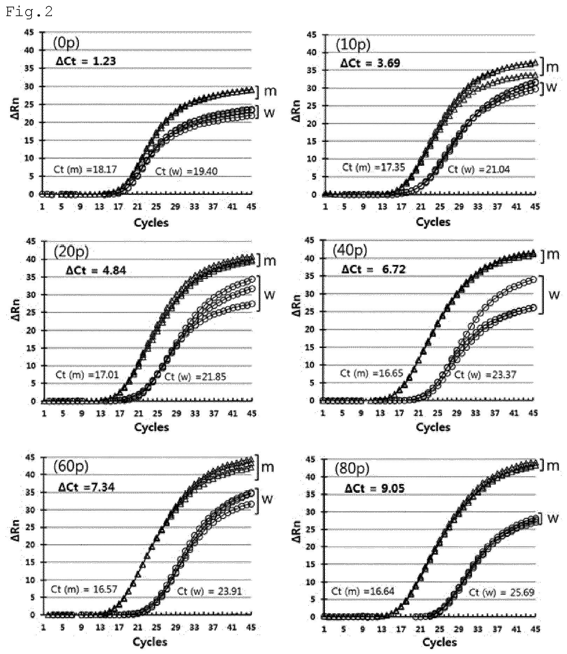

Discrimination ability was tested according to amounts of the discrimination-boosting oligonucleotide (dbOligo) (Table 2 (Test Nos. 3 and 6)). Compared to DD-type dbOligo, HD-type dbOligo allowed a low deviation over repeated tests and thus was identified to be more consistent in PCR discrimination. For both cases, the discrimination ability was improved in a dose-dependent manner over the range of 10-80 pmol in a PCR solution. ΔCt2 was measured to be up to 15.07 and maximum ΔΔCt was 13.5 (Table 2; Test No. 3 (SEQ ID NOs: 25/26) and Test No. 6 (SEQ ID NO: 13)) (FIG. 2).

### <EXAMPLE 3> Discrimination Ability for 3'-Mismatch According to Duplex Length or Tm of dbOligo

Examination was made of relationship between the duplex length or melting temperature (Tm) of duplex-forming dbOligo and 3'-mismatch discrimination was examined (Table 2 (Test Nos. 2 and 5)).

When the DD-type dbOligo was made to increase in duplex length from 20 base pairs to 30 base pairs and in Tm from 64°C to 74°C, the discrimination ability was improved from ΔCt2 = 3.04 (ΔΔCt = 1.56) to ΔCt2 = 5.70 (ΔΔCt = 4.22) (Table 2; Test No. 2 (SEQ ID NOs: 17/18, 19/20, 14/15, 21/22, 23/24, 25/26)).

Likewise, as the HD type decreased in duplex length from 24 based to 12 bases (with the resultant decrease of Tm from 68°C to 47°C) (Table 2; Test No.5 (SEQ ID NOs: 16, 27, 13, 28, 29, 30, and 31)), the discrimination ability was reduced to a ΔCt2 of 2.46 and a ΔΔCt of 0.30. When a double-stranded DNA (SEQ ID NO: 31) was endowed with a Tm (47°C) significantly lower than a PCR annealing temperature 55°C, the discrimination ability drastically decreased.

Both results indicate that the presence of a double-stranded oligonucleotide with a high Tm or with a large number of base pairs accounting for the duplex as a dbOligo improves the discrimination ability and low discrimination ability is detected in the absence of the dbOligo.

### <EXAMPLE 4> Discrimination Ability According to Sequence of Hairpin Structure dbOligo

When the non-complementary region in HD-type dbOligo, that is, the intermediate sequence that does not participate in duplex form, was arbitrarily changed, the influence attributed to kinds of nucleotides in dbOligo was not significant in the light of ΔCt2 = 6.12 (ΔΔCt = 3.85) to ΔCt2 = 7.42 (ΔΔCt = 5.15) (Table 3; Test No. 7 (SEQ ID Nos: 13, 32, 33, 34, and 35)).

In addition, when the non-complementary region in HD-type dbOligo was extended by increasing, for example, the number of adenine base from 3 to 10, the discrimination was slightly reduced with the increase of the non-complementary sequence length, but still remained higher than that of the control in the absence of dbOligo (Table 3; Test No. 8 (SEQ ID NOs: 36, 37, 38, and 39)).

In addition, a change in the kind of the complementary sequence of dbOligo resulted in a difference from ΔCt2 = 4.00 (ΔΔCt = 1.60) to ΔCt2 = 6.04 (ΔΔCt = 3.64). Even an extremely replicated sequence such as poly A/T or poly G/C brought about an improvement in discrimination although it was not significant. The data imply that the efficiency of discrimination may differ from one complementary sequence of dbOligo to another (Table 3; Test No. 9 (SEQ ID NOs: 13, 40, 41, 42, and 43)).

**TABLE 3**

| Tes t No. | Test Content | | dbOligo | | | ΔCt (Mutant Ct - Wild type Ct) | | ΔΔCt (ΔCt2 ΔCt1) |
|---|---|---|---|---|---|---|---|---|
| | | | Seq. ID | Type, * Duplex No. , ** Tm*** | Amoun t | ΔCt1^{#} | ΔCt2^{##} | |
| 7 | HD mismatch ed Seq. Species | (A) 5 | 13 | HD, 20, 64 | 20 | 2.27 | 6.12 | **3.85** |
| | | (T) 5 | 32 | | | | 6.92 | **4.65** |
| | | (C)5 | 33 | | | | 6.91 | **4.64** |
| | | (G)5 | 34 | | | | 8.97 | **6.70** |
| | | AGAGAC | 35 | | | | 7.42 | **5.15** |
| 8 | HD mismatch ed Seq. Numbers | (A) 3 | 36 | HD, 20, 64 | 20 | 1.76 | 6.16 | **4.40** |
| | | (A) 5 | 13 | | | | 6.66 | **4.90** |
| | | (A) 7 | 37 | | | | 5.72 | **3.96** |
| | | (A) 9 | 38 | | | | 4.80 | **3.04** |
| | | (A) 10 | 39 | | | | 4.43 | **2.67** |
| 9 | HD matched Seq. | random | 13 | HD, 20, 64 | 20 | 2.40 | 6.04 | **3.64** |
| | | random | 40 | HD, 20, 57 | | | 5.89 | **3.49** |
| | | random | 41 | HD, 20, 55 | | | 5.13 | **2.73** |
| | | polyA/ T | 42 | HD, 22, 38 | | | 4.00 | **1.60** |
| | | polyG/ C | 43 | HD, 20, 85 | | | 4.76 | **2.36** |

| | | | | | | | | |
|---|---|---|---|---|---|---|---|---|
| * dbOligo type: HD, hairpin structure DNA, ** Duplex no: number of bases involved in duplex formation, *** Tm: melting temperature of double-stranded oligonucleotide, # ΔCt1: ΔCt in the absence of dbOligo, ## ΔCt2: ΔCt in the presence of dbOligo. | | | | | | | | |

### <EXAMPLE 5> Discrimination Ability for 3'-Mismatch According to Template Concentration in Presence of dbOligo

When the template DNA was used in various amounts of 1 × 10⁴ copies to 1 × 10⁷ copies for targeting EGFR T790M (Table 4; Test No. 10), ΔCt1 of the control without dbOligo was insignificant (1.15 - 1.50), but ΔCt2 of the test group with dbOligo was as large as 7.53 - 8.83 (ΔΔCt 6.38 - 7.33), with no significant difference in discrimination according to amounts of template DNA. The data obtained suggest that discrimination ability for 3'-mismatch can be easily improved even in test groups using various concentrations of template DNA.

### <EXAMPLE 6> Discrimination Ability for 3'-Mismatch in ARMS PCR in Presence of dbOligo

ARMS PCR was also designed to increase discrimination for a primer with 3'-mismatch. Discrimination was examined for PCR using ARMS primers in the presence of a double-stranded oligomer (Table 4, Test No. 11). All the three types of ARMS primers (SEQ ID NOS: 2, 3, and 4) exhibited higher ΔCt1 (6.50 - 7.67), compared to AS primer (SEQ ID NO: 1), but ΔCt2 was measured to be 10.06 - 10.57 in the presence of dbOligo (SEQ ID NO: 13), implying that the double-stranded oligonucleotide, when used in the PCR, additionally improves the discrimination (ΔΔCt of 2.39 to 3.24). Taken together, the results indicate that the double-strand oligonucleotides can improve discrimination for 3'-mismatch in ARMS PCR as well as in AS PCR.

### <EXAMPLE 7> Discrimination Ability for 3'-Mismatch According to Kind of Mutant Base of Target Gene in Presence of dbOligo

There are various base mismatches of SNP in living organisms. In Examples 1- 6, discrimination ability for 3'-mismatch in the presence of dbOligo was examined by tests for targeting T790M, that is, for discriminating C and T bases (template DNA SEQ ID NOS: 44 and 45). In this Example, discrimination ability for T and G bases in EGFR L858R (temple DNA SEQ ID NOS: 46 and 47) and A and T bases in BRAF V600E (rc) (template DNA SEQ ID NO: 48, 49) were examined by real-time PCR using suitable AS primers in the presence of a double-stranded oligonucleotide. Although there was a difference in ΔCt1 according to the sequence or 3' terminal base of template DNA (T790M, 2.20; L858R, 8.14; V600E, 6.75), ΔCt2 was measured to be, 4.48 for T790M; 10.45 for L858R; and 11.43 for V600E and ΔΔCt was measured to be 2.28 for T790M; 2.31 for L858R; and 4.68 for V600E when the double-stranded oligonucleotide was added. That is, the presence of the double-stranded oligonucleotide was identified to increase the discrimination ability in all of the test groups although there are partial differences in discrimination ability according to the sequence of target genes or kind of 3'-mismatch bases (Table 4, Test No. 12).

### <EXAMPLE 8> Discrimination Ability for 3'-Mismatch According to Polymerase in Presence of dbOligo

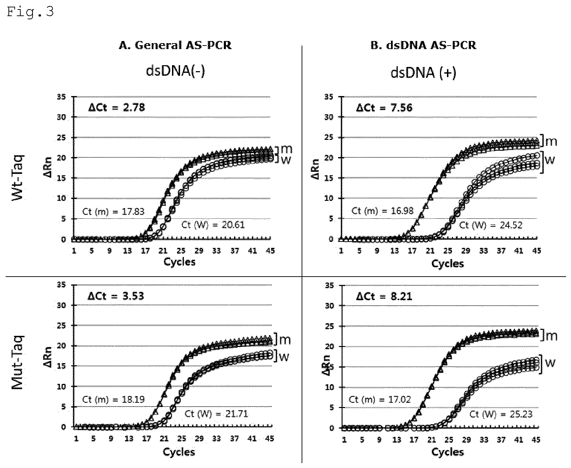

Modified polymerases may be used to increase the discrimination of PCR for 3'-mismatch. In this Example, a test for discrimination ability for 3'-mismatch was conducted using mutant (R536K) Taq DNA polymerase known to increase discrimination for 3'-mismatch (Table 4, Test No. 13; and FIG. 3). The mutant Taq DNA polymerase (Mut Taq (R536K)) slightly increased ΔCt1(= 3.53), compared to wild-type Taq DNA polymerase (Wt-Taq) (ΔCt1 = 2.78). In addition, ΔCt2 = 8.21 and ΔΔCt = 4.68 were detected in the presence of dbOligo. From this result, it was understood that although the mutant Taq DNA polymerase increased in part discrimination for 3'-mismatch, the presence of dbOligo further improved discrimination for 3'-mismatch. Particularly, dbOligo was observed to increase discrimination even for the unmodified, wild-type polymerase as measured by ΔCt1 = 2.78 for control without dbOligo and ΔCt2 = 7.56 (ΔΔCt = 4.78) for a test group with dbOligo. A high level of discrimination for 3'-mismatch was obtained only by adding dbOligo without modifying the enzyme (FIG. 3). In addition, the results indicate that the discrimination can be improved by using the double-stranded oligonucleotide for real-time PCR irrespective of wild-type polymerase or modified polymerase.

**TABLE 4**

| Tes t No. | Test Content | | dbOligo | | | ΔCt (Mutant Ct - Wild type Ct) | | ΔΔCt (ΔCt2 - ΔCt1) |
|---|---|---|---|---|---|---|---|---|
| | | | Seq ID | Type, * Duplex No,** Tm*** | Amoun t | ΔCt of dbOlig o (-) (ΔCt1) | ΔCt of dbOlig o (+) (ΔCt2) | |
| 10 | Template DNA (copies) | 1 × 10^7 | 13 | HD, 20, 64 | 80 | 1.47 | 7.98 | 6.51 |
| | | 1 × 10^6 | | | | 1.15 | 7.53 | 6.38 |
| | | 1 × 10^5 | | | | 1.31 | 8.46 | 7.15 |
| | | 1 × 10^4 | | | | 1.50 | 8.83 | 7.33 |
| 11 | ARMS primer (Seq. ID) | 2 | 13 | HD, 20, 64 | 20 | 7.67 | 10.06 | 2.39 |
| | | 3 | | | | 6.50 | 10.47 | 3.97 |
| | | 4 | | | | 7.33 | 10.57 | 3.24 |
| 12 | Detected Mutants bases (Mutant base, Wild>Mut ant base) | T790M C>T | 13 | HD, 20, 64 | 20 | 2.20 | 4.48 | 2.28 |
| | | L858R T>G | | | | 8.14 | 10.45 | 2.31 |
| | | V600E (rC ) A>T | | | | 6.75 | 11.43 | 4.68 |
| 13 | Polymera se | Taq-WT | 13 | HD, 20, 64 | 40 | 2.78 | 7.56 | 4.78 |
| | | Mut-Taq (R536K) | | | | 3.53 | 8.21 | 4.68 |

| | | | | | | | | |
|---|---|---|---|---|---|---|---|---|
| * dbOligo type: HD, hairpin structure DNA, ** Duplex no: number of bases involved in duplex formation, *** Tm: melting temperature of double-stranded oligonucleotide, # ΔCt1: ΔCt in the absence of dbOligo, ## ΔCt2: ΔCt in the presence of dbOligo. | | | | | | | | |

### <EXAMPLE 9> PCR in Presence of dbOligo without Hydrolysis Probe

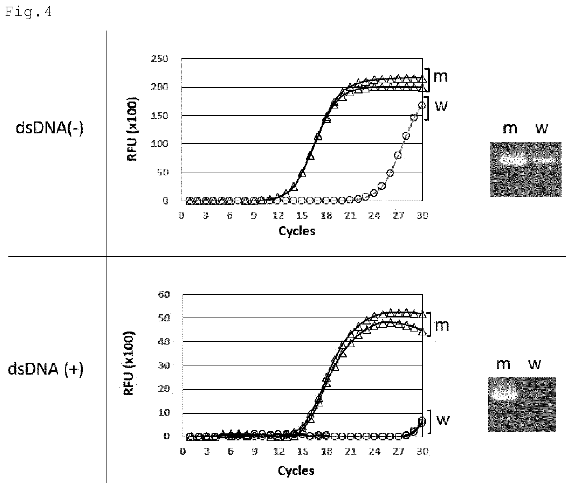

Examination was made of an improvement in discrimination for 3'-mismatch in the presence of the double-stranded oligonucleotide under the condition of using no hydrolysis probes. Using CFX96^{™} Real-Time PCR Detection System, PCR was performed in the presence of 20 pmol of the double-stranded oligonucleotide (SEQ ID NO: 13) and SYBR Green I without employing a hydrolysis probe, with the DNA of BRAF V600E (rc) serving as a template. As a result, the addition of dbOligo improved discrimination for 3'-mismatch even in the condition of using no hydrolysis probes (amplification curves in FIG. 4). In addition, the PCR product obtained by PCR without SYBR Green I was identified by agarose gel electrophoresis. The electrophoresis results showed explicit discrimination in the presence of dbOligo (electrophoresis images of FIG. 4).

**TABLE 5**

| No. | sequence | remarks |
|---|---|---|
| 1 | agccgaaggg catgagctgc a | f. primer |
| 2 | agccgaaggg catgagctac a | f. primer |
| 3 | agccgaaggg catgagctgt a | f. primer |
| 4 | agccgaaggg catgagcatc a | f. primer |
| 5 | gcatgtcaag atcacagatt ttgggcg | f. primer |
| 6 | ggacccactc catcgagatt tct | f. primer |
| 7 | agtgtggaca acccccacgt gtgc | r. primer |
| 8 | ctggctgacc taaagccacc tc | r. primer |
| 9 | cacctcagat atatttcttc atgaagac | probe |
| 10 | cggtggaggt gaggcagatg | probe |
| 11 | taccatgcag aaggaggc | probe |
| 12 | tagaccaaaa tcacctattt ttactg | probe |
| 13 | gggacagtcg gaggactcgt aaaaaacgag tcctccgact gtccc | dbOligo |
| 14 | gggacagtcg gaggactcgt ctgg | dbOligo |
| 15 | ccagacgagt cctccgactg tccc | dbOligo |
| 16 | gggacagtcg gaggactcgt ctggaaaaac cagacgagtc ctccgactgt ccc | dbOligo |
| 17 | gggacagtcg gaggactcgt | dbOligo |
| 18 | acgagtcctc cgactgtccc | dbOligo |
| 19 | gggacagtcg gaggactcgt ct | dbOligo |
| 20 | agacgagtcc tccgactgtc cc | dbOligo |
| 21 | gggacagtcg gaggactcgt ctggca | dbOligo |
| 22 | tgccagacga gtcctccgac tgtccc | dbOligo |
| 23 | gggacagtcg gaggactcgt ctggcaca | dbOligo |
| 24 | tgtgccagac gagtcctccg actgtccc | dbOligo |
| 25 | gggacagtcg gaggactcgt ctggcacagg | dbOligo |
| 26 | cctgtgccag acgagtcctc cgactgtccc | dbOligo |
| 27 | gggacagtcg gaggactcgt ctaaaaaaga cgagtcctcc gactgtccc | dbOligo |
| 28 | gggacagtcg gaggactcaa aaagagtcct ccgactgtcc c | dbOligo |
| 29 | gggacagtcg gaggacaaaa agtcctccga ctgtccc | dbOligo |
| 30 | gggacagtcg gaggaaaaac ctccgactgt ccc | dbOligo |
| 31 | gggacagtcg gaaaaaatcc gactgtccc | dbOligo |
| 32 | gggacagtcg gaggactcgt tttttacgag tcctccgact gtccc | dbOligo |
| 33 | gggacagtcg gaggactcgt cccccacgag tcctccgact gtccc | dbOligo |
| 34 | gggacagtcg gaggactcgt gggggacgag tcctccgact gtccc | dbOligo |
| 35 | gggacagtcg gaggactcgt agagcacgag tcctccgact gtccc | dbOligo |
| 36 | gggacagtcg gaggactcgt aaaacgagtc ctccgactgt ccc | dbOligo |
| 37 | gggacagtcg gaggactcgt aaaaaaaacg agtcctccga ctgtccc | dbOligo |
| 38 | gggacagtcg gaggactcgt aaaaaaaaaa cgagtcctcc gactgtccc | dbOligo |
| 39 | gggacagtcg gaggactcgt aaaaaaaaaa acgagtcctc cgactgtccc | dbOligo |
| 40 | ggagatacgt gacaggactc aaaaagagtc ctgtcacgta tctcc | dbOligo |
| 41 | gaaccctcgg taaacagaag aaaaaacttc tgtttaccga gggttc | dbOligo |
| 42 | aaaaaaaaaa aaaaaaaaaa aacccccctt tttttttttt tttttttttt | dbOligo |
| 43 | gggggggggg gggggggggg aaaaaccccc cccccccccc ccccc | dbOligo |
| 44 | cacgcacaca catatcccca tggcaaactc ttgctatccc aggagcgcag accgcatgtg 60 aggatcctgg ctccttatct cccctccccg tatctccctt ccctgattac ctttgcgatc 120 tgcacacacc agttgagcag gtactgggag ccaatattgt ctttgtgttc ccggacatag 180 tccaggaggc agccgaaggg catgagctgc gtgatgagct gcacggtgga ggtgaggcag 240 atgcccagca ggcggcacac gtgggggttg tccacgctgg ccatcacgta ggcttcctgg 300 agggagggag aggcacgtca gtgtggcttc gcatggtggc cagaaggagg ggcacatgga 360 ccccttccag gtgaagacgc atgaatgcga tcttgagttt caaaatacgt actcatggag 420 gaaaagctgt gcctgcaaaa gacctagc | template |
| 45 | cacgcacaca catatcccca tggcaaactc ttgctatccc aggagcgcag accgcatgtg 60 aggatcctgg ctccttatct cccctccccg tatctccctt ccctgattac ctttgcgatc 120 tgcacacacc agttgagcag gtactgggag ccaatattgt ctttgtgttc ccggacatag 180 tccaggaggc agccgaaggg catgagctgc atgatgagct gcacggtgga ggtgaggcag 240 atgcccagca ggcggcacac gtgggggttg tccacgctgg ccatcacgta ggcttcctgg 300 agggagggag aggcacgtca gtgtggcttc gcatggtggc cagaaggagg ggcacatgga 360 ccccttccag gtgaagacgc atgaatgcga tcttgagttt caaaatacgt actcatggag 420 gaaaagctgt gcctgcaaaa gacctagc | template |
| 46 | cgccagccat aagtcctcga cgtggagagg ctcagagcct ggcatgaaca tgaccctgaa 60 ttcggatgca gagcttcttc ccatgatgat ctgtccctca cagcagggtc ttctctgttt 120 cagggcatga actacttgga ggaccgtcgc ttggtgcacc gcgacctggc agccaggaac 180 gtactggtga aaacaccgca gcatgtcaag atcacagatt ttgggctggc caaactgctg 240 ggtgcggaag agaaagaata ccatgcagaa ggaggcaaag taaggaggtg gctttaggtc 300 agccagcatt ttcctgacac cagggaccag gctgccttcc cactagctgt attgtttaac | template |
| 47 | cgccagccat aagtcctcga cgtggagagg ctcagagcct ggcatgaaca tgaccctgaa 60 ttcggatgca gagcttcttc ccatgatgat ctgtccctca cagcagggtc ttctctgttt 120 cagggcatga actacttgga ggaccgtcgc ttggtgcacc gcgacctggc agccaggaac 180 gtactggtga aaacaccgca gcatgtcaag atcacagatt ttgggcgggc caaactgctg 240 ggtgcggaag agaaagaata ccatgcagaa ggaggcaaag taaggaggtg gctttaggtc 300 agccagcatt ttcctgacac cagggaccag gctgccttcc cactagctgt attgtttaac 360 | template |
| 48 | aaaatattcg ttttaagggt aaagaaaaaa gttaaaaaat ctatttacat aaaaaataag 60 aacactgatt tttgtgaata ctgggaacta tgaaaatact atagttgaga ccttcaatga 120 ctttctagta actcagcagc atctcagggc caaaaattta atcagtggaa aaatagcctc 180 aattcttacc atccacaaaa tggatccaga caactgttca aactgatggg acccactcca 240 tcgagatttc actgtagcta gaccaaaatc acctattttt actgtgaggt cttcatgaag 300 aaatatatct gaggtgtagt aagtaaagga aaacagtaga tctcattttc ctatcagagc 360 aagcattatg aagagtttag gtaagagatc taatttctat aattctgtaa tataatattc 420 tttaaaacat agtacttcat ctttcctctt agagtcaata agtatgtcta aaacaatgat 480 tagttctatt tagcctatat a | template |
| 49 | aaaatattcg ttttaagggt aaagaaaaaa gttaaaaaat ctatttacat aaaaaataag 60 aacactgatt tttgtgaata ctgggaacta tgaaaatact atagttgaga ccttcaatga 120 ctttctagta actcagcagc atctcagggc caaaaattta atcagtggaa aaatagcctc 180 aattcttacc atccacaaaa tggatccaga caactgttca aactgatggg acccactcca 240 tcgagatttc tctgtagcta gaccaaaatc acctattttt actgtgaggt cttcatgaag 300 aaatatatct gaggtgtagt aagtaaagga aaacagtaga tctcattttc ctatcagagc 360 aagcattatg aagagtttag gtaagagatc taatttctat aattctgtaa tataatattc 420 tttaaaacat agtacttcat ctttcctctt agagtcaata agtatgtcta aaacaatgat 480 tagttctatt tagcctatat a | template |

### Industrial Applicability

The PCR kit or method employing a discrimination-boosting oligonucleotide according to the present disclosure remarkably enhances specificity and sensitivity in general PCR as well as real-time PCR, thus making it possible to easily identify the position and amplification of complementary or non-complementary mutation loci. Therefore, the PCR kit or method of the present disclosure is advantageous for detecting alleles from a sample containing a mixture of trace amounts of various species and can easily detect a mutant gene even if it is present at a trace amount in a sample, finding a wide spectrum of applications in the gene examination of agricultural, aquatic, and livestock products and in the diagnostic medical field.

### Sequence Listing

The electronic file attached

<110>    Genotech Corp.

<120>    A PCR method for increasing the discrimination of allele and a
         PCR kit for increasing the discrimination of allele

<130>    Genotech-SPexS-191209

<160>    49

<170>    KoPatentIn 3.0

<210>    1
<211>    21
<212>    DNA
<213>    Artificial Sequence

<220>
<223>    forward primer


<400>    1
agccgaaggg catgagctgc a                                                21


<210>    2
<211>    21
<212>    DNA
<213>    Artificial Sequence

<220>
<223>    forward primer


<400>    2
agccgaaggg catgagctac a                                                21


<210>    3
<211>    21
<212>    DNA
<213>    Artificial Sequence

<220>
<223>    forward primer


<400>    3
agccgaaggg catgagctgt a                                                21


<210>    4
<211>    21
<212>    DNA
<213>    Artificial Sequence

<220>
<223>    forward primer


<400>    4
agccgaaggg catgagcatc a                                                21

<210>    5
<211>    27
<212>    DNA
<213>    Artificial Sequence

<220>
<223>    forward primer


<400>    5
gcatgtcaag atcacagatt ttgggcg                                          27


<210>    6
<211>    23
<212>    DNA
<213>    Artificial Sequence

<220>
<223>    forward primer


<400>    6
ggacccactc catcgagatt tct                                              23


<210>    7
<211>    24
<212>    DNA
<213>    Artificial Sequence

<220>
<223>    reverse primer


<400>    7
agtgtggaca acccccacgt gtgc                                             24


<210>    8
<211>    22
<212>    DNA
<213>    Artificial Sequence

<220>
<223>    reverse primer


<400>    8
ctggctgacc taaagccacc tc                                               22


<210>    9
<211>    28
<212>    DNA
<213>    Artificial Sequence

<220>
<223>    reverse primer


<400>    9
cacctcagat atatttcttc atgaagac                                         28

<210>	10
<211>	20
<212>	DNA
<213>	Artificial Sequence

<220>
<223>	hydrolysis probe


<400>	10
cggtggaggt gaggcagatg	20


<210>	11
<211>	18
<212>	DNA
<213>	Artificial Sequence

<220>
<223>	hydrolysis probe


<400>	11
taccatgcag aaggaggc	18


<210>	12
<211>	26
<212>	DNA
<213>	Artificial Sequence

<220>
<223>	hydrolysis probe


<400>	12
tagaccaaaa tcacctattt ttactg	26


<210>	13
<211>	45
<212>	DNA
<213>	Artificial Sequence

<220>
<223>	double stranded hairpin structured oligonucleotide


<400>	13
gggacagtcg gaggactcgt aaaaaacgag tcctccgact gtccc	45


<210>	14
<211>	24
<212>	DNA
<213>	Artificial Sequence

<220>
<223>	oligonucleotide which forms double strand with Seq. No. 15

<400>    14
gggacagtcg gaggactcgt ctgg                                              24


<210>    15
<211>    24
<212>    DNA
<213>    Artificial Sequence

<220>
<223>    oligonucleotide which forms double strand with Seq. No. 14


<400>    15
ccagacgagt cctccgactg tccc                                              24


<210>    16
<211>    53
<212>    DNA
<213>    Artificial Sequence

<220>
<223>    oligonucleotide which forms hairpin double strand structure


<400>    16
gggacagtcg gaggactcgt ctggaaaaac cagacgagtc ctccgactgt ccc              53


<210>    17
<211>    20
<212>    DNA
<213>    Artificial Sequence

<220>
<223>    oligonucleotide which forms double strand structure with Seq. No.
         18


<400>    17
gggacagtcg gaggactcgt                                                   20


<210>    18
<211>    20
<212>    DNA
<213>    Artificial Sequence

<220>
<223>    oligonucleotide which forms double strand structure with Seq. No.
         17


<400>    18
acgagtcctc cgactgtccc                                                   20


<210>    19
<211>    22
<212>    DNA
<213>    Artificial Sequence

<220>
<223>    oligonucleotide which forms double strand structure with Seq. No.
         20


<400>    19
gggacagtcg gaggactcgt ct                                              22


<210>    20
<211>    22
<212>    DNA
<213>    Artificial Sequence

<220>
<223>    oligonucleotide which forms double strand structure with Seq. No.
         19


<400>    20
agacgagtcc tccgactgtc cc                                              22


<210>    21
<211>    26
<212>    DNA
<213>    Artificial Sequence

<220>
<223>    oligonucleotide which forms double strand structure with Seq. No.
         22


<400>    21
gggacagtcg gaggactcgt ctggca                                          26


<210>    22
<211>    26
<212>    DNA
<213>    Artificial Sequence

<220>
<223>    oligonucleotide which forms double strand structure with Seq. No.
         21


<400>    22
tgccagacga gtcctccgac tgtccc                                          26


<210>    23
<211>    28
<212>    DNA
<213>    Artificial Sequence

<220>
<223>    oligonucleotide which forms double strand structure with Seq. No.
         24


<400>    23
gggacagtcg gaggactcgt ctggcaca                                        28

<210>    24
<211>    28
<212>    DNA
<213>    Artificial Sequence

<220>
<223>    oligonucleotide which forms double strand structure with Seq. No.
         23


<400>    24
tgtgccagac gagtcctccg actgtccc                                          28


<210>    25
<211>    30
<212>    DNA
<213>    Artificial Sequence

<220>
<223>    oligonucleotide which forms double strand structure with Seq. No.
         26


<400>    25
gggacagtcg gaggactcgt ctggcacagg                                        30


<210>    26
<211>    30
<212>    DNA
<213>    Artificial Sequence

<220>
<223>    oligonucleotide which forms double strand structure with Seq. No.
         25


<400>    26
cctgtgccag acgagtcctc cgactgtccc                                        30


<210>    27
<211>    49
<212>    DNA
<213>    Artificial Sequence

<220>
<223>    oligonucleotide which forms hairpin structure


<400>    27
gggacagtcg gaggactcgt ctaaaaaaga cgagtcctcc gactgtccc                   49


<210>    28
<211>    41
<212>    DNA
<213>    Artificial Sequence

<220>

<223>    oligonucleotide which forms hairpin structure

<400>    28
gggacagtcg gaggactcaa aaagagtcct ccgactgtcc c                41

<210>    29
<211>    37
<212>    DNA
<213>    Artificial Sequence

<220>
<223>    oligonucleotide which forms hairpin structure

<400>    29
gggacagtcg gaggacaaaa agtcctccga ctgtccc                37

<210>    30
<211>    33
<212>    DNA
<213>    Artificial Sequence

<220>
<223>    oligonucleotide which forms hairpin structure

<400>    30
gggacagtcg gaggaaaaac ctccgactgt ccc                33

<210>    31
<211>    29
<212>    DNA
<213>    Artificial Sequence

<220>
<223>    oligonucleotide which forms hairpin structure

<400>    31
gggacagtcg gaaaaatcc gactgtccc                29

<210>    32
<211>    45
<212>    DNA
<213>    Artificial Sequence

<220>
<223>    oligonucleotide which forms hairpin double strand structure

<400>    32
gggacagtcg gaggactcgt tttttacgag tcctccgact gtccc                45

<210>    33
<211>    45
<212>    DNA
<213>    Artificial Sequence

<220>
<223>    oligonucleotide which forms hairpin double strand structure


<400>    33
gggacagtcg gaggactcgt cccccacgag tcctccgact gtccc                    45


<210>    34
<211>    45
<212>    DNA
<213>    Artificial Sequence

<220>
<223>    oligonucleotide which forms hairpin double strand structure


<400>    34
gggacagtcg gaggactcgt gggggacgag tcctccgact gtccc                    45


<210>    35
<211>    45
<212>    DNA
<213>    Artificial Sequence

<220>
<223>    oligonucleotide which forms hairpin double strand structure


<400>    35
gggacagtcg gaggactcgt agagcacgag tcctccgact gtccc                    45


<210>    36
<211>    43
<212>    DNA
<213>    Artificial Sequence

<220>
<223>    oligonucleotide which forms hairpin double strand structure


<400>    36
gggacagtcg gaggactcgt aaaacgagtc ctccgactgt ccc                      43


<210>    37
<211>    47
<212>    DNA
<213>    Artificial Sequence

<220>
<223>    oligonucleotide which forms hairpin double strand structure


<400>    37
gggacagtcg gaggactcgt aaaaaaaacg agtcctccga ctgtccc                   47


<210>    38
<211>    49

<212>    DNA
<213>    Artificial Sequence

<220>
<223>    oligonucleotide which forms hairpin double strand structure


<400>    38
gggacagtcg gaggactcgt aaaaaaaaaa cgagtcctcc gactgtccc                    49


<210>    39
<211>    50
<212>    DNA
<213>    Artificial Sequence

<220>
<223>    oligonucleotide which forms hairpin double strand structure


<400>    39
gggacagtcg gaggactcgt aaaaaaaaaa acgagtcctc cgactgtccc                   50


<210>    40
<211>    45
<212>    DNA
<213>    Artificial Sequence

<220>
<223>    oligonucleotide which forms hairpin double strand structure


<400>    40
ggagatacgt gacaggactc aaaaagagtc ctgtcacgta tctcc                       45


<210>    41
<211>    46
<212>    DNA
<213>    Artificial Sequence

<220>
<223>    oligonucleotide which forms hairpin double strand structure


<400>    41
gaaccctcgg taaacagaag aaaaaacttc tgtttaccga gggttc                      46


<210>    42
<211>    50
<212>    DNA
<213>    Artificial Sequence

<220>
<223>    oligonucleotide which forms hairpin double strand structure


<400>    42
aaaaaaaaaa aaaaaaaaaa aacccccctt tttttttttt tttttttttt                   50

<210>    43
<211>    45
<212>    DNA
<213>    Artificial Sequence

<220>
<223>    oligonucleotide which forms hairpin double strand structure


<400>    43
gggggggggg gggggggggg aaaaaccccc cccccccccc ccccc                45


<210>    44
<211>    448
<212>    DNA
<213>    Homo Sapiens

<400>    44
cacgcacaca catatcccca tggcaaactc ttgctatccc aggagcgcag accgcatgtg      60

aggatcctgg ctccttatct cccctccccg tatctccctt ccctgattac ctttgcgatc     120

tgcacacacc agttgagcag gtactgggag ccaatattgt ctttgtgttc ccggacatag     180

tccaggaggc agccgaaggg catgagctgc gtgatgagct gcacggtgga ggtgaggcag     240

atgcccagca ggcggcacac gtgggggttg tccacgctgg ccatcacgta ggcttcctgg     300

agggagggag aggcacgtca gtgtggcttc gcatggtggc cagaaggagg ggcacatgga     360

ccccttccag gtgaagacgc atgaatgcga tcttgagttt caaaatacgt actcatggag     420

gaaaagctgt gcctgcaaaa gacctagc                                        448


<210>    45
<211>    448
<212>    DNA
<213>    Homo Sapiens

<400>    45
cacgcacaca catatcccca tggcaaactc ttgctatccc aggagcgcag accgcatgtg      60

aggatcctgg ctccttatct cccctccccg tatctccctt ccctgattac ctttgcgatc     120

tgcacacacc agttgagcag gtactgggag ccaatattgt ctttgtgttc ccggacatag     180

tccaggaggc agccgaaggg catgagctgc atgatgagct gcacggtgga ggtgaggcag     240

atgcccagca ggcggcacac gtgggggttg tccacgctgg ccatcacgta ggcttcctgg     300

agggagggag aggcacgtca gtgtggcttc gcatggtggc cagaaggagg ggcacatgga     360

ccccttccag gtgaagacgc atgaatgcga tcttgagttt caaaatacgt actcatggag     420

gaaaagctgt gcctgcaaaa gacctagc                                        448


<210>    46
<211>    360
<212>    DNA
<213>    Homo Sapiens

<400>     46
cgccagccat aagtcctcga cgtggagagg ctcagagcct ggcatgaaca tgaccctgaa     60

ttcggatgca gagcttcttc ccatgatgat ctgtccctca cagcagggtc ttctctgttt     120

cagggcatga actacttgga ggaccgtcgc ttggtgcacc gcgacctggc agccaggaac     180

gtactggtga aaacaccgca gcatgtcaag atcacagatt ttgggctggc caaactgctg     240

ggtgcggaag agaaagaata ccatgcagaa ggaggcaaag taaggaggtg gctttaggtc     300

agccagcatt ttcctgacac cagggaccag gctgccttcc cactagctgt attgtttaac     360

                                                                      360

<210>     47
<211>     360
<212>     DNA
<213>     Homo Sapiens

<400>     47
cgccagccat aagtcctcga cgtggagagg ctcagagcct ggcatgaaca tgaccctgaa     60

ttcggatgca gagcttcttc ccatgatgat ctgtccctca cagcagggtc ttctctgttt     120

cagggcatga actacttgga ggaccgtcgc ttggtgcacc gcgacctggc agccaggaac     180

gtactggtga aaacaccgca gcatgtcaag atcacagatt ttgggcgggc caaactgctg     240

ggtgcggaag agaaagaata ccatgcagaa ggaggcaaag taaggaggtg gctttaggtc     300

agccagcatt ttcctgacac cagggaccag gctgccttcc cactagctgt attgtttaac     360

                                                                      360

<210>     48
<211>     501
<212>     DNA
<213>     Homo Sapiens

<400>     48
aaaatattcg ttttaagggt aaagaaaaaa gttaaaaaat ctatttacat aaaaaataag     60

aacactgatt tttgtgaata ctgggaacta tgaaaatact atagttgaga ccttcaatga     120

ctttctagta actcagcagc atctcagggc caaaaattta atcagtggaa aaatagcctc     180

aattcttacc atccacaaaa tggatccaga caactgttca aactgatggg acccactcca     240

tcgagatttc actgtagcta gaccaaaatc acctattttt actgtgaggt cttcatgaag     300

aaatatatct gaggtgtagt aagtaaagga aaacagtaga tctcattttc ctatcagagc     360

aagcattatg aagagtttag gtaagagatc taatttctat aattctgtaa tataatattc     420

tttaaaacat agtacttcat ctttcctctt agagtcaata agtatgtcta aaacaatgat     480

tagttctatt tagcctatat a                                               501

<210>    49
<211>    501
<212>    DNA
<213>    Homo Sapiens

<400>    49
aaaatattcg ttttaagggt aaagaaaaaa gttaaaaaat ctatttacat aaaaaataag        60

aacactgatt tttgtgaata ctgggaacta tgaaaatact atagttgaga ccttcaatga       120

ctttctagta actcagcagc atctcagggc caaaaattta atcagtggaa aaatagcctc       180

aattcttacc atccacaaaa tggatccaga caactgttca aactgatggg acccactcca       240

tcgagatttc tctgtagcta gaccaaaatc acctattttt actgtgaggt cttcatgaag       300

aaatatatct gaggtgtagt aagtaaagga aaacagtaga tctcattttc ctatcagagc       360

aagcattatg aagagtttag gtaagagatc taatttctat aattctgtaa tataatattc       420

tttaaaacat agtacttcat ctttcctctt agagtcaata agtatgtcta aaacaatgat       480

tagttctatt tagcctatat a                                                 501

## Claims

1. A PCR kit for detection of a mutant, the kit comprising:
(a) a forward primer and a reverse primer for at least one template including a target DNA sequence having a potential mutation locus;
(b) a DNA polymerase for DNA polymerization from the forward primer and the reverse primer which combine with the template; and
(c) a discrimination-boosting oligonucleotide which is complementary to none of the template, the forward primer, and the reverse primer, can each reversibly combine with the DNA polymerase, and each form, in part or in entirety, a duplex.

2. The PCR kit of claim 1, further comprising:
(d) at least one template including a target DNA sequence having a potential mutation locus.

3. The PCR kit of claim 1, wherein a first base at the 3' terminus of the forward primer corresponds to the potential mutation locus of the target DNA sequence.

4. The PCR kit of claim 1, wherein the forward primer is an allele-specific (AS) primer or an amplification refractory mutation system (ARMS) primer.

5. The PCR kit of claim 1, wherein the discrimination-boosting oligonucleotide is at least one selected from among
a DNA duplex,
an RNA/DNA hybrid duplex,
a double-stranded oligonucleotide,
a partially or entirely complementary oligonucleotide single strand(s) capable of forming a partial or entire DNA duplex,
a partially or entirely complementary oligonucleotide single strand(s) capable of forming a partial or entire DNA/RNA hybrid duplex,
a partially or entirely complementary oligonucleotide single strand(s) capable of forming a partial or entire double-stranded oligonucleotide, and
an oligonucleotide capable of forming a partial or perfect hairpin duplex.

6. The PCR kit of claim 1, wherein the discrimination-boosting oligonucleotide includes arbitrary sequence.

7. The PCR kit of claim 1, wherein the mutation in the target DNA sequence is a single nucleotide polymorphism.

8. The PCR kit of claim 1, wherein the DNA polymerase is a thermostable DNA polymerase.

9. The PCR kit of claim 8, wherein the DNA polymerase is a wild-type or a mutant DNA polymerase.

10. The PCR kit of claim 1, wherein the discrimination-boosting oligonucleotide has a length of 10 to 100 bases (both inclusive).

11. The PCR kit of claim 1, wherein the discrimination-boosting oligonucleotide has a length of 15 to 50 bases (both inclusive).

12. The PCR kit of claim 1, wherein the discrimination-boosting oligonucleotide has a Tm identical or greater than an annealing temperature set forth for PCR amplification.

13. The PCR kit of claim 1, wherein the discrimination-boosting oligonucleotide has a Tm of 50-85°C.

14. The PCR kit of claim 1, further comprising a fluorescence resonance energy transfer probe modified with a reporter and a quencher.

15. A method for detection of a genetic mutation, the method comprising the steps of:
(a) providing a template including a target DNA sequence having a potential mutation locus, a forward primer and a reverse primer both combining with the template, a DNA polymerase polymerizing DNA from the forward primer and the reverse primer, a discrimination-boosting oligonucleotides that is complementary to none of the template, the forward primer, and the reverse primer, can reversibly combine with the DNA polymerase, and can form a partial or entire a duplex;
(b) performing a polymerase chain reaction using the DNA polymerase in the presence of the template, the forward primer, the reverse primer, and the discrimination-boosting oligonucleotide; and
(c) acquiring an amplification curve from the reaction of step (b).

16. The method of claim 15, further comprising a step of (d) determining from the amplification curve acquired in step (c) whether the target DNA sequence includes a mutation.

17. The method of claim 15, wherein a first base at the 3' terminus of the forward primer corresponds to the potential mutation locus of the target DNA sequence.

18. The method of claim 15, wherein the forward primer is an allele-specific (AS) primer or an amplification refractory mutation system (ARMS) primer.

19. The method of claim 15, wherein the discrimination-boosting oligonucleotide is at least one selected from among
a DNA duplex,
an RNA/DNA hybrid duplex,
a double-stranded oligonucleotide,
a partially or entirely complementary oligonucleotide single strand(s) capable of forming a partial or entire DNA duplex,
a partially or entirely complementary oligonucleotide single strand(s) capable of forming a partial or entire DNA/RNA hybrid duplex,
a partially or entirely complementary oligonucleotide single strand(s) capable of forming a partial or entire double-stranded oligonucleotide, and
an oligonucleotide capable of forming a partial or perfect hairpin duplex.

20. The method of claim 15, wherein the discrimination-boosting oligonucleotide includes arbitrary sequence.

21. The method of claim 15, wherein, the mutation in the target DNA sequence is a single nucleotide polymorphism.

22. The method of claim 15, wherein the DNA polymerase is a thermostable DNA polymerase.

23. The method of claim 15, wherein the DNA polymerase is a wild-type or a mutant DNA polymerase.

24. The method of claim 15, wherein the discrimination-boosting oligonucleotide has a length of 10 to 100 bases (both inclusive).

25. The method of claim 15, wherein the discrimination-boosting oligonucleotide has a length of 15 to 50 bases (both inclusive).

26. The method of claim 15, wherein the discrimination-boosting oligonucleotide has a Tm identical or greater than an annealing temperature set forth for PCR amplification.

27. The method of claim 15, wherein the discrimination-boosting oligonucleotide has a Tm of 50-85°C.

28. The method of claim 15 or 16, further comprising employing a fluorescence resonance energy transfer probe modified with a reporter and a quencher in steps (a) or (b).
